# EUROPEAN PATENT APPLICATION

(11) **EP 1 181 928 A1**
(43) Date of publication of application: **27.02.2002**
(21) Application number: 00931610.0
(22) Date of filing: 30.05.2000
(51) Int. Cl.: A61K 9/127

(54) **MICROLIPOSOMES AND PROCESS FOR PRODUCING THE SAME**

(30) Priority: 01.06.1999 JP 15357999
(71) Applicant: ONO PHARMACEUTICAL CO., LTD., Osaka-shi, Osaka 541-8526 (JP)
(72) Inventor: NAKANISHI, Chikashi, Yokohama-shi, Kanagawa 244-0803 (JP); TANI, Hidetoshi, Minase Res. Institute, ONO Pharma, Mishima-gun, Osaka 618-8585 (JP); NISHIURA, Akio, Minase Res. Institute, ONO Pharma., Mishima-gun, Osaka 618-8585 (JP)
(74) Representative: Henkel, Feiler, Hänzel
(86) International application number: JP0003472
(87) International publication number: WO0072824

(57) **Abstract**

The invention relates to microliposomes obtained by injecting a lipid-containing liposome material into an aqueous saccharide solution or saccharide slurry having a high concentration, and a production process thereof. According to the present invention, microliposomes can be easily and efficiently produced while minimizing the amount of the organic solvent employed without any severe physical means.

## Description

### TECHNICAL FIELD

The invention relates to liposomes produced by using saccharides and a production process thereof. More particularly, the invention relates to microliposomes produced by using saccharides at a high concentration and a production process thereof.

### BACKGROUND ART

Liposomes, which are closed lipid vesicles, have been employed to intentionally deliver a drug enclosed therein toward a definite site in the body and to ensure the stability or sustained release of a drug.

The particle size of liposomes is an important factor in the dynamics thereof *in vivo* and closely relates to, for example, disappearance from the blood circulatory system, distribution in various tissues, absorption in organs and migration among tissues. In order to smoothly carry out these phenomena, it is preferable to use small liposome particles. By decreasing the particle size, it also becomes possible to sterilize liposomes by filtration as required in injections. Namely, the particle size is an important factor in injections.

Many of conventional methods for producing liposomes comprise a first step of hydrating and swelling a phospholipid in an aqueous disperse medium and a second step of miniaturizing the liposomes to give a desired particle size. Various attempts have been made in each step.

In the first step of one of these conventional methods, for example, lipid is dissolved in a volatile organic solvent, the solvent is distilled from the solution to form a thin film made of the lipid, *etc.* on the container wall and then an aqueous disperse medium is added to thereby hydrate and swell the lipid film under stirring. Although this process can be carried out relatively easily, the liposomes obtained after the hydration and swelling are multi-layered vesicles having a relatively large particle size, i.e., from several to several hundred microns. In case where a smaller particle size is needed as in injections, *etc.,* it is therefore needed to employ another procedure of miniaturizing the liposomes.

Examples of the procedures for miniaturizing liposomes or uniformizing the particle size distribution in the second step include extrusion with the use of a polycarbonate filter, high-pressure emulsification and ultrasonic irradiation (see *Liposome* (Nankodo, 1988)). When the hydrated liposomes have a large particle size before the miniaturization, it is further necessary to repeatedly pass the liposome dispersion through a polycarbonate filter of the same pore size (see Japanese Patent No. 2,537,186 (WO8600238, EP185756)) or to prolong the high-pressure emulsification or ultrasonic irradiation time. In these cases, there arises the problem of a need for a prolonged production time or an enlarged production apparatus.

Accordingly, some methods have been proposed for producing microliposomes more conveniently than the above-described methods or producing microliposomes without any organic solvents.

As well known examples, (1) a surfactant removal method and an organic solvent injection method may be exemplified (see *Liposome* (Nankodo, 1988), *Liposome Experimental Manual in Life Science* (Springer-Verlag Tokyo, 1992)).

The surfactant-removal method has been known as a method for obtaining microliposomes by solubilizing a lipid with a surfactant and then removing the surfactant. However, it is necessary to use an apparatus for removing the surfactant to produce a large amount of microliposomes by using this method. In this case, the treatment time therefor becomes a problem.

The organic solvent injection method has been known as a method for producing microliposomes which can be easily hydrated by dissolving lipid in a water-soluble organic solvent (ethanol, *etc.*) and injecting the thus obtained solution into an aqueous disperse medium. The particle size of the liposomes obtained by this method largely depends on the concentration of the lipid solution. Accordingly, a smaller microliposomes can be obtained by lowering the concentration of the lipid solution. However, it is unavoidable to use a larger amount of the organic solvent in decreasing the concentration of the lipid solution.

In these methods, moreover, it is required to remove the surfactant or the organic solvent by dialysis, gel filtration or the like. Therefore, these production methods cannot be regarded as efficient.

(2) As methods of hydrating lipids without any organic solvents, it has been known to promote the hydration of lipids by using physical means, for example, heating, applying of a shear force or powdering.

However, the heating method, wherein lipid, a drug, an aqueous disperse medium and other materials are heated to the phase transition temperature of the lipid or higher under stirring (see JP-B-4-36735 or JP-B-4-28412), is inadequate for liposomes containing drugs which are unstable to heat. On the other hand, the mechanochemical method, wherein a shear force is applied to lipid, a drug, an aqueous disperse medium and other materials, suffers from a problem that a constant yield can be hardly achieved in case of fat-soluble drugs. In the spray-drying method wherein lipid and other materials are once dissolved and then powdered (see JP-B-4-37731), hydration can quickly proceed due to the amorphism and enlarged surface are of the lipid. However, this method cannot be easily put into industrial use because of the problems in heating and yield and the need for a large-scaled apparatus.

As discussed above, each of these conventional methods suffers from various problems. In addition, the miniaturization after the hydration can be hardly achieved to the satisfactory extent.

On the other hand, water-soluble additives such as saccharides and electrolytes are frequently used in producing liposomes.

The addition of these components aims at (1) obtaining the isotonicity as injections, (2) retaining the liposomal shape in freeze-drying, or (3) providing cores in forming thin lipid films.

For example, JP-A-9-110828 (EP758645) discloses that saccharides (lactose, mannitol, *etc.*) can be added as adjuvants other than the active ingredient and a 10% maltose solution was used in the production (Example 4).

In *Liposome Technology* (ed. by Gregory Gregoriadis, Vol. 1, 2nd edition, 229-252 (1993)), effects of various saccharides on the shape retention of liposomes in freeze-drying are discussed.

Moreover, JP-B-3-62696 discloses that the characteristics of liposome preparations can be improved by freeze-drying with the use of saccharides as fillers. It is also disclosed that the saccharide concentration in the production ranges from 1 to 10%.

In JP-B-5-51338 (EP119020), a method of coating a water-soluble granular carrier with a liposome film is disclosed and sorbitol, mannitol, xylitol, naturally occurring amino acids, lactose, dextrose and sucrose are exemplified therein as the carrier. It is also disclosed that an aqueous isotonic solution of a concentration of about 1 to 10% W/V (preferably from about 3 to 7% W/V) should be prepared.

As discussed above, although it has been a practice to add saccharides in producing liposomes, the saccharides are not added in order to produce microliposomes. Moreover, the saccharides are not added at such a high concentration as in the invention but at about 10% which is isotonic.

### DISCLOSURE OF THE INVENTION

An object of the invention is to provide a process for efficiently producing microliposomes while minimizing the amount of the organic solvent employed without any severe physical means, and the microliposomes obtained by this production process.

In order to overcome the above-described problems, the inventors have conducted intensive studies. As a result, they have found that microliposomes can be easily and efficiently produced by using a saccharide at such a high concentration as not employed in the prior art in the process of producing liposomes, thus completing the invention.

Accordingly, the invention provides microliposomes and a production process thereof as described below.
(1) A microliposome obtained by injecting a lipid-containing liposome material into an aqueous saccharide solution or saccharide slurry having a high concentration.
(2) The microliposome according to (1), which is obtained by injecting a lipid-containing liposome material into an aqueous saccharide solution or saccharide slurry having a high concentration, and has a particle size of smaller than 400 nm.
(3) The microliposome according to (1), wherein the aqueous saccharide solution or saccharide slurry comprises 1 to 30 parts by weight of water per 10 parts by weight of the saccharide.
(4) The microliposome according to (3), wherein the aqueous saccharide solution or saccharide slurry comprises 2 to 15 parts by weight of water per 10 parts by weight of the saccharide.
(5) The microliposome according to (1), wherein the saccharide is one or at least two selected from monosaccharides, disaccharides and sugar alcohols.
(6) The microliposome according to (5), wherein the saccharide is one or at least two selected from glucose, fructose, galactose, maltose, lactose, sucrose, trehalose, xylitol, mannitol and erythritol.
(7) The microliposome according to (1), wherein the lipid-containing liposome material comprises 5 parts by weight or less of lipid per 10 parts by weight of the saccharide.
(8) The microliposome according to (1), wherein the lipid-containing liposome material is a solution of a water-soluble organic solvent containing lipid.
(9) The microliposome according to (8), wherein the water-soluble organic solvent is selected from lower alcohols having up to 4 carbon atoms, acetone and acetonitrile.
(10) The microliposome according to (8), wherein 1 to 100 parts by weight of the water-soluble organic solvent are used per 10 parts by weight of the lipid.
(11) The microliposome according to (10), wherein 0.5 to 50 parts by weight of the water-soluble organic solvent remain per 10 parts by weight of the lipid when formulated into a freeze-dried liposome preparation.
(12) The microliposome according to (10), wherein 0.5 to 30 parts by weight of the water-soluble organic solvent remain per 10 parts by weight of the lipid when formulated into a freeze-dried liposome preparation.
(13) A process for producing a microliposome, comprising injecting a lipid-containing liposome material into an aqueous saccharide solution or saccharide slurry having a high concentration, followed by mixing.
(14) The process for producing a microliposome according to (13), wherein the microliposome is obtained by injecting a lipid-containing liposome material into an aqueous saccharide solution or saccharide slurry having a high concentration, followed by mixing, and has a particle size of less than 400 nm.
(15) The process for producing a microliposome according to (13), wherein the aqueous saccharide solution or saccharide slurry comprises 1 to 30 parts by weight of water per 10 parts by weight of the saccharide.
(16) The process for producing a microliposome according to (15), wherein the aqueous saccharide solution or saccharide slurry comprises 2 to 15 parts by weight of water per 10 parts by weight of the saccharide.
(17) The process for producing a microliposome according to (13), wherein the saccharide is one or at least two saccharides selected from monosaccharides, disaccharides and sugar alcohols.
(18) The process for producing a microliposome according to (17), wherein the saccharide is one or at least two saccharides selected from glucose, fructose, galactose, maltose, lactose, sucrose, trehalose, xylitol, mannitol and erythritol.
(19) The process for producing a microliposome according to (13), wherein the lipid-containing liposome material comprises 5 parts by weight or less of lipid per 10 parts by weight of the saccharide.
(20) The process for producing a microliposome according to (13), wherein the lipid-containing liposome material is a solution of a water-soluble organic solvent containing lipid.
(21) The process for producing a microliposome according to (20), wherein the water-soluble organic solvent is selected from lower alcohols having up to 4 carbon atoms, acetone and acetonitrile.
(22) The process for producing a microliposome according to (20), wherein 1 to 100 parts by weight of the water-soluble organic solvent is used per 10 parts by weight of the lipid.
(23) The process for producing a microliposome according to (22), wherein 0.5 to 50 parts by weight of the water-soluble organic solvent remains per 10 parts by weight of the lipid in the microliposome when formulated into a freeze-dried liposome preparation.
(24) The process for producing a microliposome according to (23), wherein 0.5 to 30 parts by weight of the water-soluble organic solvent remains per 10 parts by weight of the lipid in the microliposome when formulated into a freeze-dried liposome preparation.

### DETAILED DESCRIPTION OF THE INVENTION

The invention relates to microliposomes obtained by injecting a lipid-containing liposome material into an aqueous saccharide solution or saccharide slurry having a high concentration, and a production process thereof.

According to the process of the invention, microliposomes can be efficiently obtained while minimizing the amount of the organic solvent without any severe physical means.

In producing liposomes by the invention, a saccharide is used at a high concentration. Specifically, 10 parts by weight of the saccharide is mixed with 1 to 30 parts by weight of water. It is preferable to mix 10 parts by weight of the saccharide with 2 to 15 parts by weight of water. It is still preferable to mix 10 parts by weight of the saccharide with 3 to 10 parts by weight of water. The saccharide may used in the form of a solution or a slurry.

Examples of the saccharide used in the invention include monosaccharides such as glucose, fructose and galactose; disaccharides such as maltose, lactose, sucrose and trehalose; and sugar alcohols such as xylitol, mannitol and erythritol. Each of the saccharides can be preferably used. Either one of the saccharides or a combination of at least two thereof may be used. Preferred examples include glucose, maltose, lactose, sucrose, trehalose, xylitol, and a combination thereof. It is particularly preferable to use maltose.

In the invention, the ratio of the saccharide to the lipid is not limited. It is preferable to control the amount of the lipid in the lipid-containing liposome material to 5 parts by weight or less per 10 parts by weight of the saccharide. It is still preferable to control the amount of the lipid in the liposome material to 1 part by weight or less, particularly preferably 0.0001 to 0.5 parts by weight, per 10 parts by weight of the saccharide.

Examples of the lipid used in the invention include phospholipids and glycolipids. Specific examples include yolk lecithin, soybean lecithin, phosphatidylcholine (dimyristoyl phosphatidylcholine, dipalmitoyl phosphatidylcholine, distearoyl phosphatidylcholine, *etc.*), lysophosphatidylcholine, phosphatidylglycerol (dimyristoyl phosphatidylglycerol, dipalmitoyl phosphatidylglycerol, distearoyl phosphatidylglycerol, *etc.*), phosphatidylserine, phosphatidylethanolamine, sphingomyelin, dicetyl phosphate, phosphatidic acid, and a mixture thereof. Each of the lipids can be preferably used. Preferred examples include yolk lecithin, phosphatidylcholine (dimyristoyl phosphatidylcholine, dipalmitoyl phosphatidylcholine, distearoyl phosphatidylcholine, *etc.*), phosphatidylglycerol (dimyristoyl phosphatidylglycerol, dipalmitoyl phosphatidylglycerol, distearoyl phosphatidylglycerol, etc), and a mixture thereof.

In addition to the above-described lipid, the liposome material may contain a film stabilizer such as cholesterol, *etc.* and an antioxidant such as α-tocopherol, *etc.*

It is preferable that the lipid used in the invention is added in the form of a solution (still preferably a concentrated solution) or a lipid film or lipid powder obtained by distilling off the solvent from a lipid solution. Examples of the solvent in the lipid solution include a water-soluble organic solvent. Specific examples include lower alcohols having up to 4 carbon atoms such as methanol or ethanol, acetone and acetonitrile. Each of the organic solvents can be preferably used. It is still preferable to use ethanol.

According to the process of the invention, microliposomes can be produced while minimizing the amount of the organic solvent employed. For example, 1 to 100 parts by weight, preferably 1 to 50 parts by weight, of the organic solvent is used per 10 parts by weight of the lipid.

The liposomes according to the invention can be processed to give a freeze-dried liposome preparation. In this case, a small amount of the organic solvent employed in producing the liposomes remains therein. Since microliposomes can be produced while minimizing the amount of the organic solvent employed in the invention, only a small amount of the organic solvent remains therein. When liposomes obtained by using 1 to 100 parts by weight of the organic solvent per 10 parts by weight of the lipid are freeze-dried, for example, only a small amount of 0.5 to 50 parts by weight, preferably 0.5 to 30 parts by weight, of the organic solvent remains in the freeze-dried liposome preparation.

In the invention, the physiologically active substance to be carried in the liposomes is not particularly limited. In order to carry a water-soluble physiologically active substance, for example, the physiologically active substance is preliminarily added to an aqueous saccharide solution or a saccharide slurry and then a lipid-containing liposome material is injected thereinto. In case of a hydrophobic physiologically active substance, the physiologically active substance is dissolved in an organic solvent in the smallest amount required and then mixed with a lipid-containing liposome material, followed by the injection into an aqueous saccharide solution or a saccharide slurry.

In the step of injecting the lipid-containing liposome material into the aqueous saccharide solution or saccharide slurry having a high concentration and mixing, the mixture may be stirred if necessary. Stirring can be performed by a conventional manner or using an emulsifying/dispersing apparatus such as a homogenizer. Needless to say, a small particle size can be quickly achieved using an apparatus accelerating emulsification or dispersion.

Microliposomes can be obtained without any severe physical treatments by using the process according to the invention. For example, microliposomes having a particle size (measured by the dynamic light scattering method, *etc.*) of about 400 nm or less can be obtained. It is also possible to easily obtain liposomes having a particle size of 300 nm or less. The microliposomes thus obtained can be diluted, if necessary.

Although the liposomes produced by the process of the invention have a small particle size sufficient in practice, the liposomes may be subjected to a physical treatment such as extrusion with a polycarbonate filter, high-pressure emulsification or ultrasonic irradiation so as to further decrease the particle size or achieve more uniform particle size distribution.

### INDUSTRIAL APPLICABILITY

The liposomes containing a physiologically active substance obtained according to the invention are formulated into injections for parenteral administration such as liposome injections or solid injections to be dissolved or suspended before use. The injections may further contain stabilizers, suspending aids, emulsifiers, soothing agents, buffers, preservatives and the like. The preparations are sterilized at the final stage or produced by aseptic procedures. It is also possible that an aseptic solid product (for example, a freeze-dried product) is produced and sterilized or dissolved in aseptic distilled water for injection or other solvents before use.

Examples of other preparations containing the liposomes for parenteral administration include solutions for external use, ointments, liniments, vapors, sprays, suppositories and pessaries for intravaginal administration.

### BEST MODE FOR CARRYING OUT THE INVENTION

Now, the invention will be described in greater detail by reference to the following Examples. However, it is to be understood that the invention is not construed as being restricted thereto.

### Example 1

Maltose (1 kg) was added to water (1 L) and dissolved therein by heating. The resulting 50% aqueous maltose solution was cooled to room temperature and a lecithin solution containing yolk lecithin (0.5 g), ethanol (1.0 g) and a fat-soluble drug (PGE1•(dodecanoyloxy)ethyl ester; 0.05 g) was injected thereinto. The resulting solution was stirred at 8,000 rpm for 30 minutes to thereby give liposomes according to the invention. The liposomes were diluted with water to give a total volume of 10 L. The average particle size of the liposomes estimated by the dynamic light scattering method was 190 nm.

### Example 2

Maltose (1 kg) was added to water (1 L) and dissolved therein by heating. The resulting 50% aqueous solution of maltose was cooled to room temperature and a lecithin solution comprising yolk lecithin (5.0 g), ethanol (8.0 g) and a fat-soluble drug (PGE1•(dodecanoyloxy)ethyl ester; 0.5 g) was injected thereinto. The obtained solution was stirred at 10,000 rpm for 30 minutes to thereby give liposomes according to the invention. The liposomes were diluted with water to give a total volume of 10 L. The average particle size of the liposomes estimated by the dynamic light scattering method was 180 nm.

### Example 3

Maltose (2.5 kg) was added to water (2.5 L) and dissolved therein by heating. The resulting 50% aqueous maltose solution cooled to room temperature and a lecithin solution containing yolk lecithin (12 g), ethanol (20 g) and a fat-soluble drug (PGE1•(dodecanoyloxy)ethyl ester; 1 g) was injected thereinto. The resulting solution was stirred at 8,000 rpm for 30 minutes to thereby give liposomes according to the invention. The liposomes were diluted with water to give a total volume of 25 L. The average particle size of the liposomes estimated by the dynamic light scattering method was 210 nm.

### Example 4

Into a maltose slurry obtained by adding maltose (1 kg) to water (0.3 L), a lecithin solution containing yolk lecithin (5.0 g) and ethanol (8.0 g) was injected. The slurry was stirred at 8,000 rpm for 15 minutes to thereby give liposomes according to the invention. The liposomes were diluted with water to give a total volume of 10 L. The average particle size of the liposomes estimated by the dynamic light scattering method was 200 nm.

### Example 5

Into a maltose slurry obtained by adding maltose (20 g) to water (8 ml), a solution containing dimyristoyl phosphatidylcholine (0.1 g) and ethanol (0.2 g) was injected. The slurry was stirred at 100 rpm for 60 minutes to thereby give liposomes according to the invention. The liposomes were diluted with water to give a total volume of 200 ml. The average particle size of the liposomes estimated by the dynamic light scattering method was 130 nm.

### Example 6

Into a maltose slurry obtained by adding maltose (20 g) to water (8 ml), a solution containing a mixture (0.2 g) of dipalmitoyl phosphatidylcholine, dipalmitoyl phosphatidylglycerol and cholesterol (65:5:30) and ethanol (0.3 g) was injected. The slurry was stirred at 100 rpm for 60 minutes to thereby give liposomes according to the invention. The liposomes were diluted with water to give a total volume of 200 ml. The average particle size of the liposomes estimated by the dynamic light scattering method was 220 nm.

### Comparative Example

Into a 10% aqueous maltose solution obtained by adding maltose (10 g) to water (90 ml), a lecithin solution containing yolk lecithin (0.3 g) and ethanol (3.0 g) was injected. The solution was stirred at 100 rpm for 60 minutes to thereby give liposomes according to the invention. The liposomes were diluted with water to give a total volume of 100 ml. The average particle size of the liposomes estimated by the dynamic light scattering method was 800 nm.

### Example 7

Into 50% aqueous solutions (20 ml) of various saccharides, a lecithin solution containing yolk lecithin (0.6 g) and ethanol (0.8 g) was injected. Each solution thus obtained was stirred at 100 rpm for 24 hours to thereby give liposomes according to the invention. The liposomes were diluted with water to give a total volume of 100 ml. Then the absorbance of the diluted solution at 400 nm was measured and the turbidity was calculated. The results are shown in Table 1.

**Table 1**

| Saccharide | Turbidity |
|---|---|
| Maltose | 1.03 |
| Trehalose | 1.10 |
| Lactose | 0.51 |
| Sucrose | 0.97 |
| Glucose | 0.81 |
| Xylitol | 0.91 |

A turbidity is a value which is employed as an index for particle size. The smaller the turbidity is, the smaller the particle size is (ed. by Gregory Gregoriadis, *LIPOSOME TECHNOLOGY*, Vol. 1, 2nd edition, 568-571 (1993)).

Considering the results in Table 1 together with the results in Example 1 using maltose, it can be understood that liposomes having particle sizes similar to or smaller than the particle size obtained by using maltose could be obtained.

## Claims

1. A microliposome obtained by injecting a lipid-containing liposome material into an aqueous saccharide solution or saccharide slurry having a high concentration.

2. The microliposome according to claim 1, which is obtained by injecting a lipid-containing liposome material into an aqueous saccharide solution or saccharide slurry having a high concentration, and has a particle size of smaller than 400 nm.

3. The microliposome according to claim 1, wherein the aqueous saccharide solution or saccharide slurry comprises 1 to 30 parts by weight of water per 10 parts by weight of the saccharide.

4. The microliposome according to claim 3, wherein the aqueous saccharide solution or saccharide slurry comprises 2 to 15 parts by weight of water per 10 parts by weight of the saccharide.

5. The microliposome according to claim 1, wherein the saccharide is one or at least two selected from monosaccharides, disaccharides and sugar alcohols.

6. The microliposome according to claim 5, wherein the saccharide is one or at least two selected from glucose, fructose, galactose, maltose, lactose, sucrose, trehalose, xylitol, mannitol and erythritol.

7. The microliposome according to claim 1, wherein the lipid-containing liposome material comprises 5 parts by weight or less of lipid per 10 parts by weight of the saccharide.

8. The microliposome according to claim 1, wherein the lipid-containing liposome material is a solution of a water-soluble organic solvent containing lipid.

9. The microliposome according to claim 8, wherein the water-soluble organic solvent is selected from lower alcohols having up to 4 carbon atoms, acetone and acetonitrile.

10. The microliposome according to claim 8, wherein 1 to 100 parts by weight of the water-soluble organic solvent are used per 10 parts by weight of the lipid.

11. The microliposome according to claim 10, wherein 0.5 to 50 parts by weight of the water-soluble organic solvent remain per 10 parts by weight of the lipid when formulated into a freeze-dried liposome preparation.

12. The microliposome according to claim 10, wherein 0.5 to 30 parts by weight of the water-soluble organic solvent remain per 10 parts by weight of the lipid when formulated into a freeze-dried liposome preparation.

13. A process for producing a microliposome, comprising injecting a lipid-containing liposome material into an aqueous saccharide solution or saccharide slurry having a high concentration, followed by mixing.

14. The process for producing a microliposome according to claim 13, wherein the microliposome is obtained by injecting a lipid-containing liposome material into an aqueous saccharide solution or saccharide slurry having a high concentration, followed by mixing, and has a particle size of less than 400 nm.

15. The process for producing a microliposome according to claim 13, wherein the aqueous saccharide solution or saccharide slurry comprises 1 to 30 parts by weight of water per 10 parts by weight of the saccharide.

16. The process for producing a microliposome according to claim 15, wherein the aqueous saccharide solution or saccharide slurry comprises 2 to 15 parts by weight of water per 10 parts by weight of the saccharide.

17. The process for producing a microliposome according to claim 13, wherein the saccharide is one or at least two saccharides selected from monosaccharides, disaccharides and sugar alcohols.

18. The process for producing a microliposome according to claim 17, wherein the saccharide is one or at least two saccharides selected from glucose, fructose, galactose, maltose, lactose, sucrose, trehalose, xylitol, mannitol and erythritol.

19. The process for producing a microliposome according to claim 13, wherein the lipid-containing liposome material comprises 5 parts by weight or less of lipid per 10 parts by weight of the saccharide.

20. The process for producing a microliposome according to claim 13, wherein the lipid-containing liposome material is a solution of a water-soluble organic solvent containing lipid.

21. The process for producing a microliposome according to claim 20, wherein the water-soluble organic solvent is selected from lower alcohols having up to 4 carbon atoms, acetone and acetonitrile.

22. The process for producing a microliposome according to claim 20, wherein 1 to 100 parts by weight of the water-soluble organic solvent is used per 10 parts by weight of the lipid.

23. The process for producing a microliposome according to claim 22, wherein 0.5 to 50 parts by weight of the water-soluble organic solvent remains per 10 parts by weight of the lipid in the microliposome when formulated into a freeze-dried liposome preparation.

24. The process for producing a microliposome according to claim 23, wherein 0.5 to 30 parts by weight of the water-soluble organic solvent remains per 10 parts by weight of the lipid in the microliposome when formulated into a freeze-dried liposome preparation.
